# EUROPÄISCHE PATENTSCHRIFT

(11) **EP 0 623 573 B1**
(45) Veröffentlichungstag und Bekanntmachung des Hinweises auf die Patenterteilung: **27.08.1997**
(21) Anmeldenummer: 94105982.6
(22) Anmeldetag: 18.04.1994
(51) Int. Cl.: C07C 25/02, C07C 25/08, C07C 17/00

(54) **Verfahren zur Herstellung von m-Chloraromaten**
Process for the preparation of m-chloro-aromatics
Procédé pour la préparation de composés m-chloroaromatiques

(30) Priorität: 30.04.1993 DE 4314299
(43) Veröffentlichungstag der Anmeldung: 09.11.1994
(73) Patentinhaber: BAYER AG, 51368 Leverkusen (DE)
(72) Erfinder: Pies, Dr. Michael, D-47249 Duisburg (DE); Fiege, Dr. Helmut, D-51373 Leverkusen (DE); Puppe, Dr. Lothar, D-51399 Burscheid (DE); Käsbauer, Dr. Josef, D-42929 Wermelskirchen (DE)

(56) Entgegenhaltungen:
- EP-A- 0 072 008
- EP-A- 0 278 729

## Beschreibung

Die vorliegende Erfrindung betrifft ein besonders vorteilhaftes Verfahren zur Herstellung von m-Chloraromaten durch Isomerisierung entsprechender o- und/oder p-Chloraromaten an Zeolithen.

Dichlorbenzol und Chlortoluol werden im allgemeinen durch Chlorierung von Monochlorbenzol bzw. Toluol hergestellt. Dabei werden, in Abhängigkeit von den angewendeten Reaktionsbedingungen und Katalysatoren, unterschiedlich zusammengesetzte Isomerenmischungen erhalten, die o-, m- und p-Dichlorbenzol bzw. o-, m- und p-Chlortoluol enthalten. Das jeweilige m-Isomere liegt in solchen Mischungen jedoch stets in sehr geringen Mengen vor (siehe Ullmanns Encyclopedia of Industrial Chemistry, 5. Auflage, Band A6, Seiten 333 ff.). Für einige Anwendungen, z.B. für die Herstellung von Zwischenprodukten für Pflanzenschutz- und Arzneimitteln werden jedoch hauptsächlich m-Dichlorbenzol und m-Chlortoluol benötigt.

Es sind deshalb Verfahren zur Herstellung von m-Dichlorbenzol und m-Chlortoluol bekannt geworden, bei denen man die entsprechenden o- und/oder p-Verbindungen isomerisiert. Von Interesse sind dabei insbesondere Verfahren, die Zeolithe als Isomerisierungskatalysatoren anwenden, da sie verfahrenstechnisch und ökologisch vorteilhaft sind (siehe z.B. DE-A 33 34 673, DE-A 34 20 706, EP-A 46 665 und JP-Anm. 57-27 279 = JP-OS 58-144 330). Nachteilig bei diesem Verfahren ist jedoch, daß die Zeolithe innerhalb von Zeiträumen von längstens ca. 200 Stunden regeneriert werden müssen.

Dies trifft auch auf Verfahren zu, bei denen ein Zusatz von Wasserstoff zu den zu isomerisierenden Chloraromaten möglich ist. So beschreibt die DE-A33 34 673 ein Verfahren zur Herstellung von m-Dichlorbenzol durch Isomerisierung von o- und/oder p-Dichlorbenzol, bei dem man beim Arbeiten in der Gasphase zur Herabsetzung des Partialdrucks der Dichlorbenzole gegebenenfalls Verdünnungsmittel, wie Stickstoff oder Wasserstoff zufügen kann (siehe Seite 6, Zeilen 4 bis 7). Konkret beschrieben ist ein Zusatz von 4 und 5,35 Molen Stickstoff pro Mol eingesetzte Dichlorbenzole (siehe Beispiele 1 und 4). Über die Zeiträume, nach denen die Zeolithkatalysatoren regeneriert werden müssen, wird keine Aussage gemacht.

Die DE-A 34 20 706 beschreibt ein Verfahren zur Isomerisierung von 2-, 3- und/oder 4-Chlortoluol an zirkonhaltigen Zeolithen, die sich durch besonders große Aktivität bei langer Standzeit auszeichnen sollen. Man kann in der gasförmigen oder flüssigen Phase arbeiten und gegebenenfalls dem Einsatzmaterial Wasserstoff zumischen, z.B. in Mengen von 880 Mol%, bezogen auf 2-Chlortoluol. Die Aktivität und Selektivität des Katalysators hält 210 Stunden an (siehe Beispiel 1b). Bei bis dahin bekanntgewordenen Verfahren findet schon bei deutlich kürzeren Zeiträumen eine Katalysatordesaktivierung statt (siehe Seite 4, Zeilen 9 bis 12).

Schließlich ist aus der EP-A 46 665 ein Isomerisierungsverfahren von halogenierten Toluolen bekannt, bei dem man saure Zeolithe als Katalysatoren einsetzt. Das Verfahren kann laut Beschreibung gegebenenfalls in Gegenwart von Wasserstoff durchgeführt werden (siehe Seite 4, Zeilen 14 bis 16). In Beispiel 1, dem einzigen Beispiel mit Gaszusatz, werden jedoch 5 Mol Stickstoff pro Mol o-Chlortoluol -Chlorbenzol- Gemisch eingesetzt. Es fehlen quantitative Angaben über die Zeiträume, nach denen die Katalysatoren regeneriert werden müssen.

Es besteht daher immer noch ein Bedürfnis nach einem Isomerisierungsverfahren zur Herstellung von m-Chloraromaten, bei dem eine hohe Katalysatoraktivität über Zeiträume von mehr als ca. 200 Stunden erhalten bleibt, um so den Aufwand für die Regenerierung der Katalysatoren zu vermindern.

Es wurde nun ein Verfahren zur Herstellung von m-Chloraromaten der Formel (I) 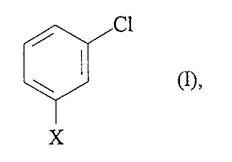 in der
- X: für Methyl oder Chlor steht,
durch Isomerisierung von o- und/oder p-Chloraromaten der Formeln (IIa) und (IIb) 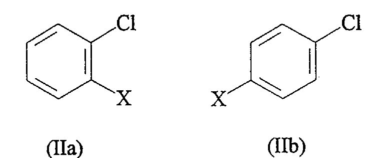 in denen
- X: für Methyl oder Chlor stehen,
gefunden, daß dadurch gekennzeichnet ist, daß man o- und/oder p-Chloraromaten der Formeln (IIa) und (IIb) bei 150 bis 500°C in flüssiger Phase und in Gegenwart von 1 bis 30 Mol-% Wasserstoff, bezogen auf eingesetzte Chloraromaten, mit Zeolithen in Kontakt bringt.

In das erfindungsgemäße Verfahren kann man technisch reines o- oder p-Dichlorbenzol oder technisch reines o- oder p-Chlortoluol oder beliebige o- und/oder p-Dichlorbenzol und/oder o- und/oder p-Chlortoluole enthaltende Gemische einsetzen. Bevorzugt setzt man Gemische ein, wie sie bei der Dichlorierung von Benzol oder der Monochlorierung von Toluol oder bei der Aufarbeitung solcher Chlorierungsgemische anfallen, z.B. bei der Aufarbeitung durch fraktionierte Destillation und/oder fraktionierte Kristallisation. Solche Gemische können z.B. neben o- und/oder p-Dichlorbenzol oder o- und/oder p-Chlortoluol gegebenenfalls Anteile an Ausgangsprodukten für die Chlorierung, also Benzol oder Toluol, an minderchlorierten Produkten, also Monochlorbenzol, an höherchlorierten Produkten, also z.B. Tri- und Tetrachlorbenzol oder z.B. Di- und Trichlortoluol, sowie bereits z.B. bis maximal 20 Gew.-% m-Dichlorbenzol oder z.B. bis zu maximal 15 Gew.-% m-Chlortoluol enthalten. Vorzugsweise enthalten die eingesetzten Chloraromaten weniger als 8 Gew.-% des jeweiligen m-Isomeren.

Für das erfindungsgemäße Verfahren ist es ohne besondere Bedeutung, woher die eingesetzten Chloraromaten oder Chloraromatengemische stammen und wieviel o- und/oder p-Isomere sie enthalten.

Geeignete Temperaturen für das erfindungsgemäße Verfahren sind solche im Bereich von 150 bis 500°C. Bevorzugt arbeitet man bei 200 bis 450°C. Der Druck ist bei der jeweiligen Temperatur mindestens so hoch zu wählen, daß die eingesetzten Chloraromaten im wesentlichen in flüssiger Phase vorliegen. Das bedeutet, daß man schon bei Reaktionstemperaturen von weniger als 200°C bei erhöhtem Druck arbeiten muß. Geeignete Drucke können, je nach der angewendeten Reaktionstemperatur, z.B. im Bereich 1 bis 100 bar liegen. Vorzugsweise liegen sie im Bereich von 10 bis 50 bar.

Als Reaktoren kommen beispielsweise druckfeste Reaktionsrohre in Frage, die mit stückigen Zeolithen oder Zeolith-Granulaten gefüllt sind.

Beim Reaktormaterial handelt es sich vorzugsweise um Nickel oder Tantal bzw. um Legierungen mit hohen Anteilen (z.B. Werkstoff-Nr. 24 602, nach DIN 17 742 (1963)) dieser Metalle.
Ungeeignet sind Reaktormaterialien mit z.B. hohem Eisengehalt.

Es ist ein wesentliches Merkmal der vorliegenden Erfindung, daß man in Gegenwart von 1 bis 30 Mol-% Wasserstoff, bezogen auf eingesetzte Chloraromaten, arbeitet. Vorzugsweise beträgt diese Menge 3 bis 10 Mol-%, insbesondere 3,5 bis 8 Mol-%. Zu hohe Wasserstoffmengen sind zu vermeiden, da sonst die Verweilzeit am Zeolith-Katalysator negativ beeinflußt wird und die Isomerisierung nicht mehr praktisch vollständig in der flüssigen Phase ablaufen kann.

In das erfindungsgemäße Verfahren können verschiedene Zeolithe als Katalysatoren eingesetzt werden, beispielsweise solche vom Pentasil-, Mordenit- und/oder Faujasit-Typ. Bevorzugt werden vom Pentasiltyp ZSM-5-Zeolithe eingesetzt, besonders bevorzugt ZSM-5-Zeolithe in der H-Form.

Es ist zweckmäßig, den Zeolith in granulierter Form, beispielsweise in Form von Teilchen mit einem mittleren Korndurchmesser von 1 bis 8 mm einzusetzen. Gegebenenfalls können die Zeolithe übliche Binder enthalten.

Man kann das erfindungsgemäße Verfahren beispielsweise so durchführen, daß man die Zeolithe mit 0,05 bis 5 h⁻¹ (WHSV) belastet. Vorzugsweise wählt man die Belastung im Bereich 0,07 bis 5 h⁻¹ (WHSV), besonders bevorzugt im Bereich 0,1 bis 2,5 h⁻¹ (WHSV).

Falls die zum Einsatz in das erfindungsgemäße Verfahren vorgesehenen o- und/oder p-Chloraromaten störende Verunreinigungen in fester und/oder gelöster Form enthalten, ist es vorteilhaft, diese Chloraromaten z.B. durch Filtration und/oder eine Behandlung mit Kieselerde, Bleicherde oder ähnlichen Mitteln zu reinigen, bevor man sie mit Zeolithen in Kontakt bringt.

Mit dem erfindungsgemäßen Verfahren gelingt es, Zeolith-Katalysatoren bei der Isomerisierung von o- und p-Chloraromaten über längere Zeiträume bei hohen Aktivitäten zu halten. Häufig ist dann aus wirtschaftlichen Gründen eine Regeneration der Zeolithe nicht mehr sinnvoll, sondern eher der Einsatz einer frischen Charge. Dadurch wird die Häufigkeit und die Dauer von Produktionsunterbrechungen auf ein sehr viel niedrigeres Niveau gebracht als bisher für möglich gehalten wurde.

Es ist ausgesprochen überraschend, daß diese Laufzeitverlängerung von Zeolithen bei der Isomerisierung von o- und p-Chloraromaten in flüssiger Phase mit so geringen Wasserstoff-Zusätzen erzielt werden kann. Bisher wurden, wenn überhaupt, nur Wasserstoff-Zusätze von mehreren Mol pro Mol Einsatzmaterial in Betracht gezogen und Laufzeiten von maximal ca. 200 Stunden erzielt. Ein Zusammenhang zwischen Laufzeit und Wasserstoffzusatz ist bisher nicht erkannt worden. Auf erfindungsgemäße Weise können bei guter Aktivität Laufzeiten von beispielsweise 1000 bis 1500 Stunden realisiert werden. Eine dann zu beobachtende Aktivitätsabnahme kann durch eine geringe Erhöhung der Reaktionstemperatur, bespielsweise um 5 bis 20°C, ausgeglichen werden (siehe Beispiel 2).

### Beispiele

### Beispiel 1

Ein Gemisch aus 34 Gew.-% o-, 2 Gew.-% m- und 58,2 Gew.-% p-Dichlorbenzol sowie 5,3 Gew.-% Trichlorbenzolen wurde unter Zusatz von 5 Mol-% Wasserstoff, bezogen auf die Summe der Dichlorbenzole, an H-ZSM-5-Zeolith isomerisiert.

Die Umsetzung wurde bei 350°C und 30 bar sowie einer Belastung (WHSV) von 0,1 pro Stunde in der Flüssigphase durchgeführt.

Die Zusammensetzung des Umsetzungsproduktes wurde nach 24 Stunden und dann ungefähr alle 100 weiteren Stunden gaschromatographisch untersucht. Einzelheiten sind in der Tabelle 1 zusammengestellt.

Man ersieht daraus, daß nach knapp 1000 Stunden das isomerisierte Gemisch einen praktisch unverändert hohen Anteil (40 Gew.-%) an m-Dichlorbenzol enthielt. Das entspricht einer Katalysatorstandzeit, die mindestens rund 5 mal länger ist als die längste bisher bekannt gewordene Katalysatorstandzeit.

### Vergleichsbeispiel 1

Es wurde verfahren wie in Beispiel 1, jedoch wurde kein Wasserstoff zugesetzt. Einzelheiten sind ebenfalls in der Tabelle 1 zusammengestellt. Man ersieht daraus, daß in Abwesenheiten von Wasserstoff die Katalysatoraktivität bereits nach rund 230 Stunden um über 20 % abgenommen hat.

In Tabelle 1 ist in der Spalte "Gehalt an m-Dichlorbenzol" angegeben wieviel Gew.-% m-Dichlorbenzol nach der jeweils angegebenen Zeit im Reaktionsgemisch enthalten war.

### Beispiel 2

Es wurde verfahren wie in Beispiel 1 beschrieben, jedoch wurde eine andere Charge H-ZSM-5-Zeolith eingesetzt und die Umsetzung 1500 Stunden lang durchgeführt. Nach einer Laufzeit von 1450 Stunden wurde die Temperatur auf 360°C erhöht.

Einzelheiten sind in der Tabelle 1 zusammengestellt. Auch nach 1500 Stunden wurde (wieder) ein Isomerengemisch erhalten, das über 40 Gew.-% m-Dichlorbenzol enthielt.

**Tabelle 1**

| Beispiel 1 | | Vergleichsbeispiel 1 | | Beispiel 2 | |
|---|---|---|---|---|---|
| Zeit (h) | Gehalt an m-Dichlorbenzol (%) | Zeit (h) | Gehalt an m-Dichlorbenzol (%) | Zeit (h) | Gehalt an m-Dichlorbenzol (%) |
| 24 | 42,5 | 27 | 42,2 | 31 | 45,9 |
| 121 | 42,8 | 70 | 42 | 122 | 45,8 |
| 214 | 42,0 | 179 | 37,9 | 196 | 43,7 |
| 312 | 40,6 | 191 | 35,9 | 349 | 42,6 |
| 408 | 41,1 | 233 | 33,3 | 415 | 39,6 |
| 504 | 41,1 | | | 504 | 40,3 |
| 611 | 40,1 | | | 595 | 39,9 |
| 699 | 39,0 | | | 665 | 39,5 |
| 815 | 38,3 | | | 780 | 39,4 |
| 914 | 39,4 | | | 871 | 39,5 |
| 986 | 40,0 | | | 986 | 39,5 |
| | | | | 1114 | 38,2 |
| | | | | 1210 | 37 |
| | | | | 1409* | 35,9 |
| | | | | 1501 | 41,1 |

| | | | | | |
|---|---|---|---|---|---|
| *) nach 1450 Stunden Temperatur auf 360°C erhöht. | | | | | |

### Beispiel 3

Es wurde analog Beispiel 1 verfahren, jedoch wurde bei 300 C gearbeitet und reines o-Chlortoluol isomerisiert. Einzelheiten sind in der Tabelle 2 zusammengefaßt. Man ersieht daraus, daß nach über 600 Stunden im Reaktionsgemisch ein praktisch unveränderter Anteil an m-Chlortoluol enthalten war.

Gemäß dem Stand der Technik (DE-A 34 20 706) enthält das Reaktionsprodukt trotz laufend ansteigender Temperatur nach 210 Stunden nur 16,4 Gew.-% m-Chlortoluol (siehe dort Tabelle 1, Selektivität 3-Chlortoluol in Bezug gesetzt zu Umsatz 2-Chlortoluol).

**Tabelle 2**

| Beispiel 3 | |
|---|---|
| Zeit (h) | Gehalt an m-Chlortoluol (%) |
| 48 | 27,6 |
| 116 | 28,9 |
| 199 | 29,1 |
| 311 | 29,9 |
| 403 | 28,1 |
| 500 | 29,2 |
| 613 | 29,1 |

## Patentansprüche

1. Verfahren zur Herstellung von m-Chloraromaten der Formel (I) 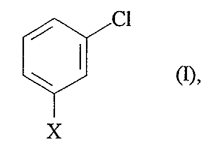 in der
X für Methyl oder Chlor steht,
durch Isomerisierung von o- und/oder p-Chloraromaten der Formeln (IIa) und (IIb) 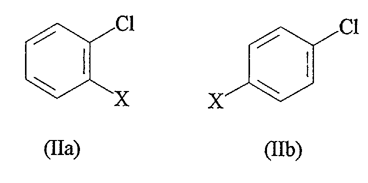 in der
X für Methyl oder Chlor stehen,
dadurch gekennzeichnet, daß man o- und/oder p-Chloraromaten der Formeln (IIa) und/oder (IIb) bei 150 bis 500°C in flüssiger Phase und in Gegenwart von 1 bis 30 Mol-% Wasserstoff, bezogen auf eingesetzte Chloraromaten, mit Zeolithen in Kontakt bringt.

2. Verfahren nach Anspruch 1, dadurch gekennzeichnet, daß man technisch reines o- oder p-Dichlorbenzol oder technisch reines o- oder p-Chlortoluol oder beliebige o- und/oder p-Dichlorbenzol und/oder o- und/oder p-Chlortoluol enthaltende Gemische einsetzt.

3. Verfahren nach Ansprüchen 1 und 2, dadurch gekennzeichnet, daß man bei Drucken arbeitet, bei denen die eingesetzten Chloraromaten im wesentlichen in flüssiger Phase vorliegen.

4. Verfahren nach Ansprüchen 1 bis 3, dadurch gekennzeichnet, daß man bei Drucken im Bereich 10 bis 100 bar arbeitet.

5. Verfahren nach Ansprüchen 1 bis 4, dadurch gekennzeichnet, daß man in Gegenwart von 3 bis 10 Mol-% Wasserstoff, bezogen auf eingesetzte Chloraromaten, arbeitet.

6. Verfahren nach Ansprüchen 1 bis 5, dadurch gekennzeichnet, daß man als Zeolithe solche vom Pentasil-, Mordenit- und/oder Faujasit-Typ einsetzt.

7. Verfahren nach Ansprüchen 1 bis 6, dadurch gekennzeichnet, daß man ZSM-5-Zeolithe in der H-Form einsetzt.

8. Verfahren nach Ansprüchen 1 bis 6, dadurch gekennzeichnet, daß man es mit einer Belastung der Zeolithe im Bereich 0,05 bis 5 h⁻¹ (WHSV) durchführt.

## Claims

1. Process for preparing m-chloroaromatics of the formula (I) 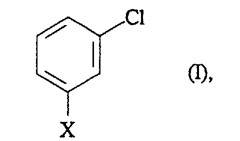 in which
X represents methyl or chlorine,
by isomerizing o- and/or p-chloroaromatics of the formulae (IIa) and (IIb) 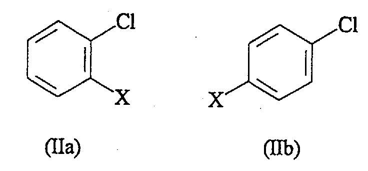 in which
X represents methyl or chlorine,
characterized in that o- and/or p-chloroaromatics of the formulae (IIa) and/or (IIb) are brought into contact with zeolites at from 150 to 500°C in the liquid phase and in the presence of from 1 to 30 mol% of hydrogen, based on the chloroaromatics used.

2. Process according to Claim 1, characterized in that technical grade o- or p-dichlorobenzene or technical grade o- or p-chlorotoluene or any desired mixtures containing o- and/or p-dichlorobenzene and/or o-and/or p-chlorotoluene is used.

3. Process according to Claims 1 and 2, characterized in that it is carried out at pressures at which the chloroaromatics used are present essentially in the liquid phase.

4. Process according to Claims 1 to 3, characterized in that it is carried out at pressures in the range from 10 to 100 bar.

5. Process according to Claims 1 to 4, characterized in that it is carried out in the presence of from 3 to 10 mol% of hydrogen, based on the chloroaromatics used.

6. Process according to Claims 1 to 5, characterized in that the zeolites used are those of the pentasil, mordenite and/or faujasite type.

7. Process according to Claims 1 to 6, characterized in that ZSM-5 zeolites in the H form are used.

8. Process according to Claims 1 to 6, characterized in that it is carried out at a weight hourly space velocity over the zeolites in the range from 0.05 to 5 h⁻¹.

## Revendications

1. Procédé de préparation de m-chloroaromatiques de formule (1) 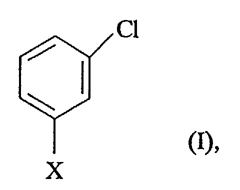 où
X représente un méthyle ou un chlore,
par isomérisation de o- et/ou p-chloroaromatiques de formules (IIa) et (IIb). 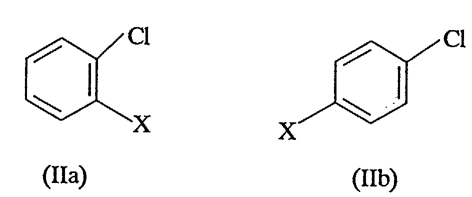 où X représente un méthyle ou un chlore,
caractérisé en ce qu'on met en contact des o- et/ou p-chloroaromatiques de formules (IIa) et/ou (IIb) entre 150 et 500°C en phase liquide et en présence de 1 à 30 % molaires d'hydrogène par rapport aux chloroaromatiques mis en oeuvre.

2. Procédé selon la revendication 1, caractérisé en ce qu'on met en oeuvre du o- ou p-dichlorobenzène techniquement pur ou du o- ou p-chlorotoluène techniquement pur ou n'importe quel mélange contenant du o-et/ou du p-dichlorobenzène et/ou du o- et/ou du p-chlorotoluène.

3. Procédé selon les revendications 1 et 2, caractérisé en ce qu'on travaille sous des pressions auxquelles les chloroaromatiques mis en oeuvre se présentent essentiellement en phase liquide.

4. Procédé selon les revendications 1 à 3, caractérisé en ce qu'on travaille à des pressions comprises entre 10 et 100 bar.

5. Procédé selon les revendications 1 à 4, caractérisé en ce qu'on travaille en présence de 3 à 10 % molaires d'hydrogène par rapport aux chloroaromatiques mis en oeuvre.

6. Procédé selon les revendications 1 à 5, caractérisé en ce qu'on utilise comme zéolithes celles du type pentasile, mordénite et/ou faujasite.

7. Procédé selon les revendications 1 à 6, caractérisé en ce qu'on utilise des zéolithes ZSM-5 sous forme H.

8. Procédé selon les revendications 1 à 6, caractérisé en ce qu'on réalise une charge des zéolithes dans l'intervalle de 0,005 à 5 h⁻¹ (WHSV).
